# EUROPEAN PATENT SPECIFICATION

(11) **EP 2 760 028 B1**
(45) Date of publication and mention of the grant of the patent: **12.12.2018**
(21) Application number: 14152079.1
(22) Date of filing: 22.01.2014
(51) Int. Cl.: G21K 1/02, A61B 6/03, A61B 6/06, A61B 6/00, G01N 23/046, G21G 4/08, G21K 1/10

(54) **Radiation generator**
Strahlungsgenerator
Générateur de rayonnement

(30) Priority: 23.01.2013 US 201361755749 P; 15.05.2013 KR 20130055281
(43) Date of publication of application: 30.07.2014
(73) Proprietor: Samsung Electronics Co., Ltd., Gyeonggi-do 443-742 (KR)
(72) Inventor: Cho, Min-kook, Gyeonggi-do (KR); Bae, Jeong-a, Gyeonggi-do (KR); Sakai, Yukio, Gyeonggi-do (KR)
(74) Representative: Grünecker Patent- und Rechtsanwälte PartG mbB

(56) References cited:
- WO-A1-2011/128550
- AT-B- 410 148
- US-A- 5 028 789
- US-A- 5 374 974

## Description

### BACKGROUND

### 1. Field

Methods and apparatuses consistent with the exemplary embodiments relate to a radiation generator that generates radiation.

### 2. Description of the Related Art

A radiation imaging apparatus irradiates a predetermined amount of radiation onto an object, for example, onto a body or the like, and measures radiation, which has penetrated the object. The radiation is measured through a radiation detector in order to calculate a radiation absorption rate of each point of the object so as to form the radiation absorption rates as an image. In order to do so, a radiation imaging apparatus performs two-dimensional (2D) radiation imaging at various angles to obtain an image at one point.

In particular, research has been conducted with regard to a radiation generator with a simpler structure when performing radiation imaging at various angles. Also, methods of acquiring clearer radiation images have been studied.

US patent 5 028 789 teaches neutron radiography using a convergent collimator of moderator material joined coaxially to a divergent collimator made of neutron-absorbing material. Neutron-emitting radioisotopes are placed at a predetermined distance from the convergent collimator cavity to generate thermal neutrons.
International patent application WO 2011/128550 A1 discloses a photon-guiding device for a radiotherapy apparatus with a central frustum having a plurality of metal channels and an outer casing enclosing microchannels wherein the channels and microchannels have a frusto-conical shape and a common apex.

### SUMMARY

The foregoing and/or other aspects may be achieved by providing a radiation generator that generates radiation, an anti-scatter grid that removes scattered radiation, and a radiation imaging apparatus including at least one of the radiation generator and the anti-scatter grid.

An exemplary embodiment provides a radiation generator having a simple structure.

The present disclosure also provides an anti-scatter grid that removes scattered radiation.

The present disclosure also provides a radiation imaging apparatus including at least one of the radiation generator and the anti-scatter grid.

According to an aspect of the exemplary embodiment, there is provided a radiation generator according to claim 1. Preferred embodiments are subject of the dependent claims.

The radiation irradiated in the specified direction may be irradiated within a convergence angle of the converger.

The radiation source is located inside the converger, and a cross-section of the converger becomes smaller toward the first opening.

The converger includes: a first member configured to block radiation which is irradiated in a direction opposite from an object; and a second member configured to surround the first member and configured to guide radiation irradiated in a specified direction toward the object.

At least one of the first member and the second member may include a material that absorbs the irradiated radiation.

The radiation generator may further include: a collimator configured to open the first opening to control the radiation to be irradiated onto the object.

The collimator is configured to horizontally move in a direction parallel with the first opening to control an amount of radiation irradiated onto the object.

The collimator is configured to rotate based on a central axis of the radiation generator in order to control an amount of radiation irradiated onto the object.

The collimator may include a second opening configured to pass radiation which has passed through the first opening.

The collimator may include a first area having a first absorption amount with respect to the radiation and a second area having a second absorption amount, different from the first radiation absorption amount, with respect to the radiation.

In an aspect of an exemplary embodiment, the radiation source may generate X-rays.

The radiation generator further includes: a diverger which has a cross-section that increases away from the first opening and which diverges the radiation that has passed through the first opening.

In an aspect of an exemplary embodiment, radiation directed toward the object through the diverger may be irradiated within a smaller one of a convergence angle of the converger and a divergence angle of the diverger.

According to an illustrative example not being part of the invention, there is provided a radiation imaging apparatus including: a radiation generator; and a radiation detector which includes a plurality of pixels, wherein each of the plurality of pixels detect radiation that has penetrated an object.

The radiation imaging apparatus may further include: an anti-scatter grid which is located between the object and the radiation detector and configured to remove scattered radiation irradiated from the radiation source.

In an illustrative example, radiation that has passed through the anti-scatter grid may focus on the radiation generator.

The anti-scatter grid may include: a plurality of radiation paths which pass the radiation to allow the radiation to be incident onto the radiation detector; and a plurality of barriers which divide the plurality of radiation paths into a two-dimensional (2D) grid and blocks radiation travelling between the plurality radiation paths. A plurality of radiation paths may be two-dimensionally arrayed in an area corresponding to one of the pixels.

At least one of the plurality of radiation paths may be filled with a material which the radiation penetrates.

A cross-section of at least one of the plurality of radiation paths may become larger toward the radiation detector.

According to another illustrative example not being part of the invention, there is provided an anti-scatter grid which removes scattered radiation. The anti-scatter grid may include: a plurality of radiation paths which pass radiation to be incident onto pixels detecting the radiation; and a plurality of barriers which divide the plurality of radiation paths into a two-dimensional (2D) grid and block radiation travelling between the radiation paths. The plurality of radiation paths may be two-dimensionally arrayed in an area corresponding to one of the plurality of pixels.

A cross-section of at least one of the plurality of radiation paths may become larger toward the radiation detector.

At least one of the plurality of radiation paths may be filled with a material which the radiation penetrates.

### BRIEF DESCRIPTION OF THE DRAWINGS

The above and other aspects will become more apparent by describing in detail exemplary embodiments thereof with reference to the attached drawings in which:
FIG. 1 is a view illustrating an external portion of a radiation imaging apparatus according to an illustrative example not being part of the invention;
FIG. 2 is a cross-sectional view illustrating a radiation generator according to an illustrative example;
FIG. 3 is a view illustrating an operation of a collimator when a radiation imaging apparatus performs radiation imaging, according to an illustrative example;
FIG. 4 is a plan view illustrating a collimator that may be applied to the radiation generator of FIG. 2;
FIG. 5 is a plan view illustrating a collimator that may be applied to the radiation generator of FIG. 2;
FIG. 6A is a view illustrating an irradiation of radiation performed by using the collimator of FIG. 5;
FIG. 6B is a view illustrating an interruption or blocking of radiation emission performed by using the collimator of FIG. 5;
FIGS. 7A through 7C are views illustrating collimators;
FIG. 8 is a cross-sectional view illustrating a radiation generator according to an exemplary embodiment of the present invention;
FIG. 9 is a cross-sectional view illustrating a radiation generator according to another exemplary embodiment;
FIG. 10 is a schematic view illustrating a radiation detector according to an illustrative example not being part of the invention;
FIG. 11 is a view illustrating an anti-scatter grid according to an illustrative example;
FIG. 12 is a view illustrating a relationship between an anti-scatter grid and a radiation detector; and
FIG. 13 is a schematic view illustrating a relationship between a radiation generator, an anti-scatter grid, and a radiation detector according to an illustrative example.
FIG. 14 is a view enlarging a radiation path and a barrier of FIG. 11.

### DETAILED DESCRIPTION

Hereinafter, a radiation generator, an anti-scatter grid, and a radiation imaging apparatus including at least one of radiation generator and the anti-scatter grid will be described in detail with reference to the attached drawings. In the description of the drawings, the same or corresponding elements are denoted by the same reference numerals, and their repeated descriptions are omitted.

An "object" as described herein may include, for example, a human, an animal, or a part of the human or the animal. For example, the object may include an organ, such as the liver, the heart, the uterus, breasts, the abdomen, or the like, or a blood vessel. However, an object is not limited to a living object and can be, for example, luggage or a package. Also, a "user" as described herein may be a medical expert, such as a doctor, a nurse, a medical laboratory technologist, a medical imaging expert, or the like, or a technician who repairs a medical apparatus, but the present disclosure is not limited thereto.

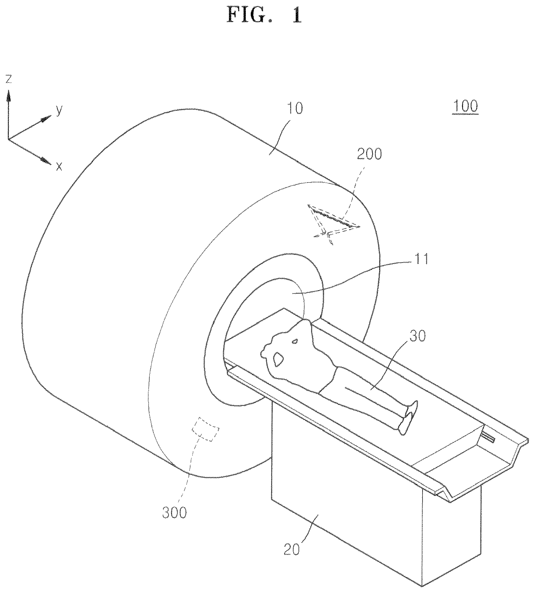

FIG. 1 is a view illustrating an external portion of a radiation imaging apparatus 100 according to an illustrative example not part of the present invention. The radiation imaging apparatus 100 is, for example, a computed tomography (CT) apparatus in FIG. 1, but the present disclosure is not limited thereto. Therefore, the radiation imaging apparatus 100 may be applied to all types of imaging apparatuses that perform imaging by using radiation.

As shown in FIG. 1, the radiation imaging apparatus 100 includes a gantry 10 and an examination table 20. The gantry 10 has a first opening 11 having a cylindrical shape in the center, and an object 30 is positioned in the first opening 11. A radiation generator 200 that irradiates or emits radiation and a radiation detector 300 that detects radiation transmitted through the object 30 are disposed inside the gantry 10. The radiation generator 200 may be located in a direction opposite of the radiation detector 300. The object 30 is positioned in a center of a predetermined area of the first opening 11 of the gantry 10. The predetermined area may be in a center of a circumference of the first opening 11. The radiation generator 200 and the radiation detector 300 are installed in the gantry 10 in a structure that allows radiation to be vertically incident on the object 30.

The gantry 10 is rotated around the object 30 at a 360° angle or at a predetermined angle by a gantry driver (not shown) to obtain an image of the object 30 at various angles by the radiation generator 200 and the radiation detector 300. The gantry driver may horizontally move back and forth, i.e., move along an x-axis, so that a part of the object 30 to be imaged is positioned in a center of the gantry 10. The gantry driver may be installed inside or outside of the gantry 10.

The examination table 20 is provided as, for example, a bed having a predetermined width on which an object is laid down and positioned. The object 30 can be, for example, a patient. Also, an examination driver (not shown) is installed in a predetermined area of the examination table 20 in the center of the first opening 11 of the gantry 10. The examination table 20 may be horizontally moved back and forth by the examination table driver, so that the part of the patient to be imaged is positioned in an internal center of the gantry 10. The examination table driver may move the examination table up and down, i.e., in a z-axis direction, or to the left and right, i.e., in a y direction, according to a body size of the patient and the part of the patient to be imaged, so as to acquire a clear image.

The radiation generator 200 generates radiation and irradiates the radiation onto the object 30. The radiation generator 200 may irradiate the radiation in a fan shape onto the object 30.

Examples of an irradiation pattern of the radiation generator 200 include a conventional irradiation (an axial irradiation), a helical irradiation, a variable pitch helical irradiation, a helical shuttle irradiation, etc. The conventional irradiation refers to a method of rotating the radiation generator 200 and the radiation detector 300 whenever the examination table 20 moves in the x-axis direction at predetermined intervals, to acquire projection data. The helical irradiation refers to a method of rotating the radiation generator 200 and the radiation detector 300 to move the examination table 20 at a predetermined speed in order to acquire projection data. The variable pitch helical irradiation refers to a method of rotating the radiation generator 200 and the radiation detector 300 to vary a speed of the examination table 20 in order to acquire projection data. The helical shuttle irradiation refers to a method of rotating the radiation generator 200 and the radiation detector 300 to accelerate and decelerate the examination table 20 in order to move the examination table 20 back and forth in positive and negative directions of the x-axis, thereby acquiring projection data. The radiation generator 200 may irradiate the radiation in one of the above-described irradiation patterns, but is not limited to these exemplary patterns.

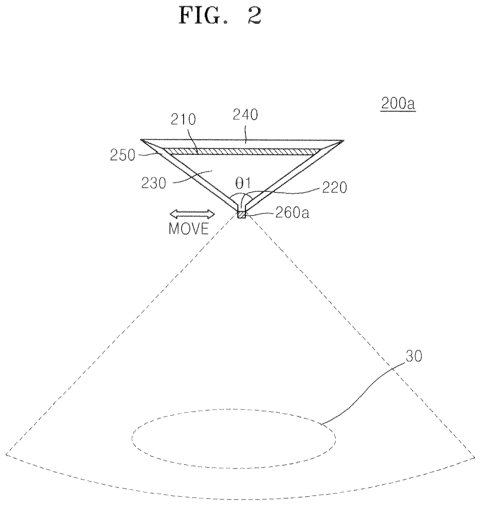

FIG. 2 is a cross-sectional view illustrating a radiation generator 200a according to an illustrative example.

Referring to FIG. 2, the radiation generator 200a includes a source 210 and a radioisotope and generates radiation, a first opening 220 through which radiation irradiated in a specified direction and through which the generated radiation passes, and a converger 230 that converges the radiation irradiated from the source 210 into the opening 220. The radiation can be emitted in a direction specified by, for example, the user of the radiation generator 200a.

The source 210 includes the radioisotope and generates the radiation. The radiation may be X-rays. However the radiation is not limited thereto and may be gamma rays. The source 210 may be symmetrical about a central axis of the radiation generator 200a. The source 210 generates the radiation in all directions. The radioisotope may include at least one of ruthenium-177, cerium-137, 141, or 143, terbium-161, holmium-166, erbium-166 or 172, thulium-172, ytterbium-169, yttrium-90, actinium-255, astatine-211, cerium-137, erbium-166, gadolinium-148, 159, holmium-166, iodine-124, titanium-45, rhodium-105, palladium-103, rhenium-186, 188, scandium-47, samarium-153, strontium-89, thulium-172, vanadium-48, ytterbium-169, yttrium-90, silver-Ill, americium-241, cadmium-109, and cobalt-57.

The source 210 that generates the radiation is disposed inside of the converger 230, and the converger 230 converges the radiation so that the radiation irradiated in specified direction from the source 210 passes through the first opening 220. A cross-section of the converger 230 becomes smaller from the source 210 toward the first opening 220. Therefore, the converger 230 interrupts or blocks the radiation which is irradiated in a different direction from the specified direction from among the generated radiation, or guides the radiation toward the specified direction. The converger 230 may be symmetrical about the central axis of the radiation generator 200a.

For example, the converger 230 includes a first member 240 that interrupts radiation which is irradiated in an opposite direction from the object 30 and a second member 250 that surrounds the first member 240 and guides the radiation to be irradiated onto the object 30 in a specified direction. The first member 240 may be disposed to contact the source 210. The first member 240 may also include a material that absorbs radiation. For example, the first member 240 may include silver (Ag), gold (Au), tungsten (W), molybdenum (Mo), or the like. Therefore, the first member 240 may absorb radiation irradiated from the source, which is coming from a direction which is opposite the object 30, from among the generated radiation.

The second member 250 may also include a material that absorbs radiation. The second member 250 may absorb radiation irradiated in a different direction from the specified direction among the generated radiation and only the radiation which is irradiated in the specified direction may pass through the second member 250.

The radiation irradiated in the specified direction may be radiation that is irradiated at an angle lower than or equal to a convergence angle θ1 of the converger 230. In other words, radiation that is irradiated from the source 210 at an angle exceeding the convergence angle θ1 may be incident onto and absorbed by the second member 250. A shape of the radiation passing through the first opening 220 may be equal to a shape of a cross-section of the first opening 220. The shape of the cross-section of the first opening 220 may be equal to a shape of a cross-section of a radiation detector 300 so as to efficiently perform radiography.

The radiation generator 200a further includes a collimator 260a that controls the radiation irradiated from the first opening 220. The collimator 260a may have a flat plate shape and may be formed of a material that absorbs radiation. Since the source 210 continuously generates radiation, the collimator 260a may close the first opening 220 to prevent radiation from being irradiated outside of the first opening 220 if radiography is not being performed. If radiography is being performed, the collimator 260a may open the first opening 220 to irradiate the radiation onto the object 30.

The collimator 260a may horizontally move in a direction parallel with the first opening 220 to open or close the first opening 220. The collimator 260a is driven by a collimator driver (not shown).

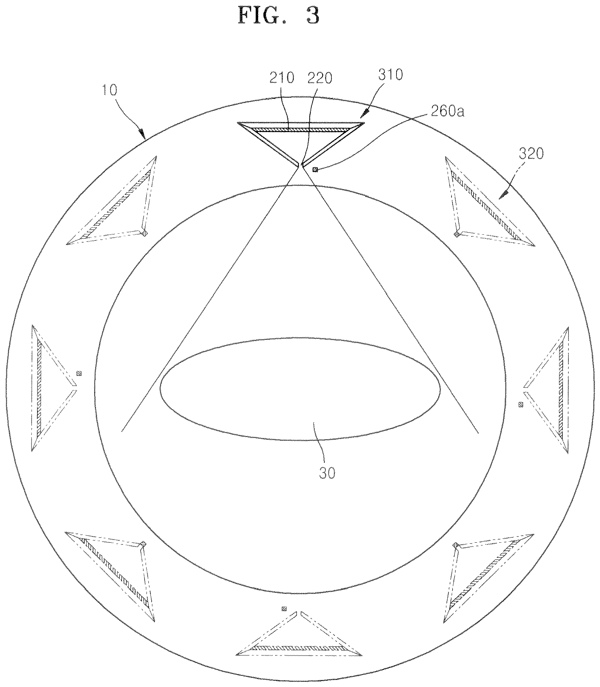

FIG. 3 is a view illustrating an operation of the collimator 260a when a radiation imaging apparatus performs radiography, according to an illustrative example.

If the radiation imaging apparatus performs radiography four times when the radiation generator 200a performs a single rotation, the radiation generator 200a may irradiate radiation onto the object 30 as shown in FIG. 3. A radiation detector (not shown) synchronizes with the irradiation of the radiation of the radiation generator 200a to detect the radiation when the radiation generator 200a irradiates the radiation. When the radiation generator 200a irradiates radiation 310, the collimator 260a may open the first opening 220. When the radiation generator 200a interrupts the irradiation of the radiation 320, the collimator 260a may horizontally move in a direction parallel with the first opening 220 to close the first opening 220.

The collimator 260a may close the whole area of the first opening 220, but the exemplary embodiment is not limited thereto. The collimator 260a may also close an area of the first opening 220. For example, the collimator 260a may selectively block an irradiation area to irradiate radiation only onto a particular interest area of the object 30. The collimator 260a may adjust an amount of radiation irradiated onto the object 30.

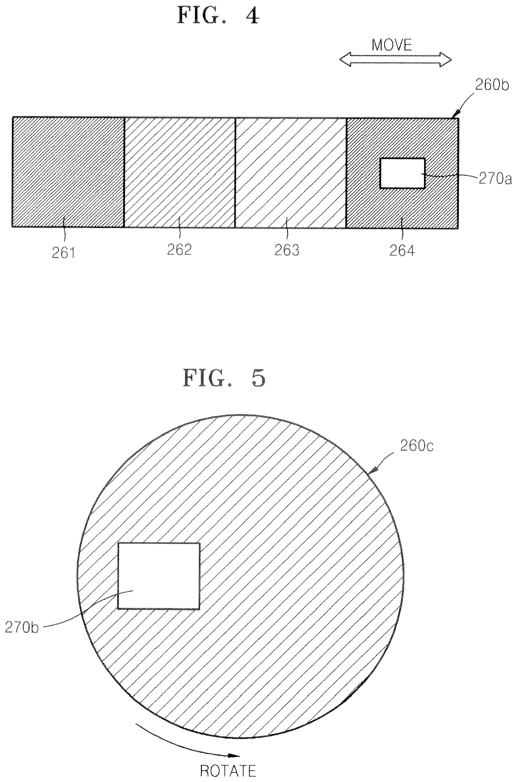

FIG. 4 is a plan view of a collimator 260b applied to the radiation generator 200a of FIG. 2, according to an exemplary embodiment of the collimator. As shown in FIG. 4, the collimator 260b is divided into a plurality of areas, namely, first area 261, second area 262, third area 263, and fourth area 264. The first area 261 may be formed of a material that completely blocks radiation, and the other areas, namely, the second area 262, third area 263, and fourth area 264, may be formed of a material that blocks some radiation. For example, the second area 262 may be formed of a material that transmits 80% of radiation, and the third area 263 may be formed of a material that transmits 50% of radiation.

Also, a second opening 270a is formed in the fourth area 264 of the collimator 260b. Radiation only passes through an area in which the second opening 270a and the first opening 220 overlap with each other, and can therefore adjust a size of a radiation area. The collimator 260b may horizontally move in a direction parallel with the first opening 220 so that one of the first area 261, second area 262, third area 263, and fourth area 264 overlap with the first opening 220, in order to adjust an amount of emitted radiation or a size of a radiation area. Alternatively, the collimator 260b may not overlap with the first opening 220 to irradiate all of radiation that has passed through the first opening 220.

Alternatively, the collimator 260b may rotate to open or close the first opening 220.

FIG. 5 is a plan view illustrating a collimator 260c according to another exemplary embodiment of the collimator.

Referring to FIG. 5, the collimator 260c is disposed between the first opening 220 and the object 30 and blocks radiation irradiated from the first opening 220. A cross-section of the collimator 260c may have a flat plate shape that is larger than a cross-section of the first opening 220 and includes an area in which a second opening 270b, through which radiation passes, is formed. Therefore, a radiation generator 200b may repeatedly perform an irradiation of radiation and an interruption of radiation due to a rotation of the collimator 260c.

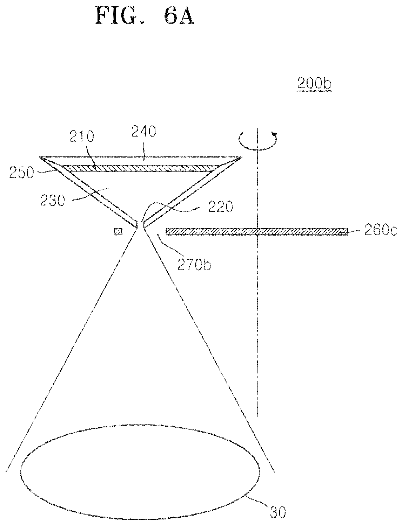

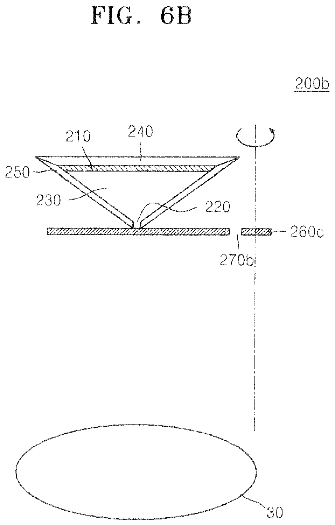

FIG. 6A is a view illustrating an irradiation of radiation performed by using the collimator 260c of FIG. 5 in radiation generator 200b. FIG. 6B is a view illustrating an interruption of radiation emission performed by using the collimator 260c of FIG. 5 to radiation generator 200b.

As shown in FIG. 6A, when the collimator 260c rotates, at least a portion of the first opening 220 and at least a portion of the second opening 270b overlap with each other. In this case, radiation passes through an area in which the first opening 220 and second opening 270b overlap with each other, to be irradiated onto the object 30.

As shown in FIG. 6B, when the collimator 260c rotates, the first opening 220 and second opening 270b do not overlap with each other. In this case, although radiation irradiated from the source 210 passes through the first opening 220, the radiation is interrupted by the collimator 260c. Therefore, the radiation generator 200b interrupts the irradiation of the radiation.

The number of openings in a collimator is not limited to one, and thus a plurality of openings may be formed, and sizes and shapes of the openings may vary. If a large number of second openings are formed, a rotation speed of a collimator may be reduced. A radiation generator can include various sizes for the second opening and can control a radiation area.

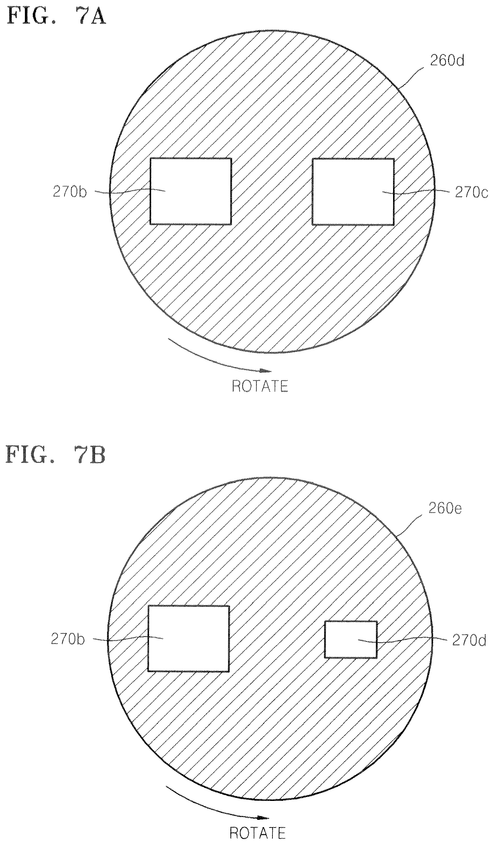

FIGS. 7A through 7C are views illustrating collimators 260d, 260e, and 260f according to various exemplary embodiments of collimators. As shown in FIG. 7A, a plurality of second openings 270b and 270c may be formed in the collimator 260d. Although two openings, namely, the second openings 270b and 270c, are shown in FIG. 7A, the exemplary embodiment is not limited to two openings. However, if a large number of second openings are formed, a rotation speed of a collimator may be reduced, and an irradiation and an interruption of radiation of a radiation generator may repeatedly occur.

As shown in FIG. 7B, the size of second opening 270b and second opening 270d of the collimator 260e may be different from each other. Therefore, if radiation is to be irradiated onto a wide radiation area, the second opening 270b which has a larger size than second opening 270d may overlap with the first opening 220. If radiation is to be irradiated onto a narrow radiation area, the collimator 260e may rotate to overlap the second opening 270d having a small size with the first opening 220.

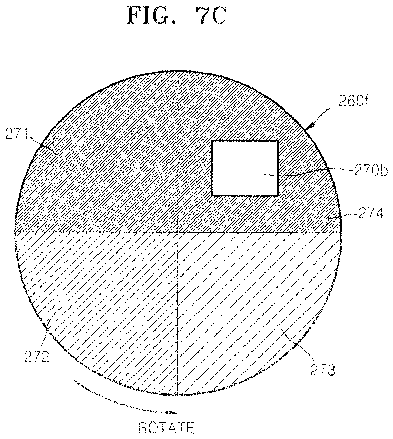

As shown in FIG. 7C, the collimator 260f may be divided into a plurality of areas, namely, first area 271, second area 272, third area 273, and fourth area 274. The areas, namely, the first area 271 and the fourth area 274, may be formed of a material that completely blocks radiation, and the second area 272 and third area 273 may be formed of a material that partially blocks radiation. For example, the second area 272 may be formed of a material that transmits 80% of radiation, and the third area 273 may be formed of a material that transmits 50% of radiation. The second opening 270b may be formed in the fourth area 274 of the collimator 260f. Therefore, radiation passes through an area in which the second opening 270b and the first opening 220 overlap with each other, to adjust a size of a radiation area. When the collimator 260f rotates, one of the first area 271, second area 272, third area 273, and fourth area 274 may overlap with the first opening 220 to adjust an amount of radiation or the radiation area.

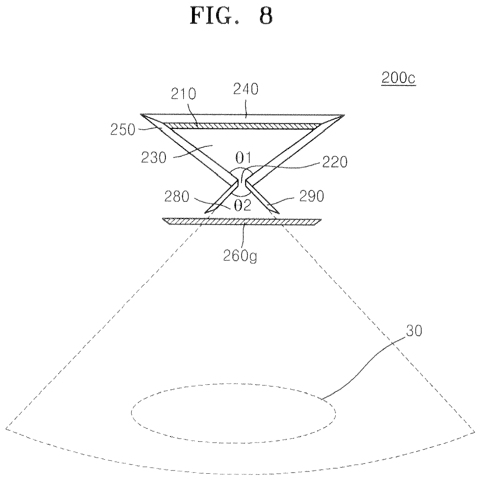

FIG. 8 is a cross-sectional view illustrating a radiation generator 200c according to an exemplary embodiment of the present invention.

Referring to FIG. 8, the radiation generator 200c includes a source 210 and a converger 230, in common with the radiation generator 200a of FIG. 2. The radiation generator 200c further includes a diverger 280 that diverges radiation that has passed through the first opening 220. As the diverger 280 extends away from the first opening 220, a cross-section of the diverger 280 becomes larger. As the radiation that has passed through the first opening 220 gets closer to the object 30, a density of the radiation becomes lower.

An end of the diverger 280 is formed by a third member 290. An end of the third member 290 is connected to the converger 230, and another end of the third member 290 is directed toward the object 30. The third member 290 may be made of or include a material that absorbs radiation. Therefore, the third member 290 may further limit an amount of radiation that has passed through the first opening 220. For example, if a divergence angle θ2 of the diverger 280 is smaller than a convergence angle θ1 of the converger 230, part of the radiation, which is irradiated from the source 210 in a direction within the convergence angle θ1 of the converger 230, passes through the first opening 220. However, since the divergence angle θ2 of the diverger 280 is smaller than the convergence angle θ1 of the converger 230, a partial radiation that has passed through the first opening 220 is interrupted by the diverger 280, and part of the radiation, which is irradiated in a direction within the divergence angle θ2 of the diverger 280, passes through the diverger 280.

Also, a shape of the radiation which is emitted may be equal to the cross-section of the first opening 220 or a cross-section of the diverger 280. The cross-section of first opening 220 or the diverger 280 may be equal to a cross-section of a radiation detector so as to efficiently perform radiography.

The converger 230 and the diverger 280 may be symmetrical to each other about the first opening 220, but are not limited thereto. The converger 230 and the diverger 280 may be asymmetrical to each other about the first opening 220. If the converger 230 and the diverger 280 are symmetrical to each other about the first opening 220, a density of radiation irradiated through the diverger 280 may be uniform.

A collimator 260g is disposed between the diverger 280 and the object 30 and controls the amount of radiation irradiated onto the diverger 280. The collimator 260g may be made of or include a material that absorbs radiation. Since the source 210 continuously generates radiation, the collimator 260g may contact the other end of the third member 290 to prevent radiation from being irradiated outside of the third member 290 if radiography is not being performed. However, if radiography is performed, the collimator 260g opens the diverger 280 so that radiation is emitted to an object 30.

The collimator 260g may interrupt all of the radiation which is emitted, but the exemplary embodiment is not limited thereto. The collimator 260g may interrupt a part of the radiation. For example, the collimator 260g may selectively interrupt a radiation area to irradiate radiation only onto a particular area of interest on the object 30. The collimator 260g may adjust an amount of the radiation irradiated onto the object 30.

The collimator 260g may have a flat plate shape and may horizontally move in a direction parallel with the first opening 220 in order to control an irradiation of radiation or may rotate based on a central axis of the radiation generator 200c to control the irradiation of the radiation. The collimator 260g applied to the radiation generator 200c of FIG. 8 may be the collimator 260a, 260b, 260d, 260e, or 260f described above. However, since the collimator 260g opens or closes the diverger 280, a size of the collimator 260g and a size of an opening (not shown) may be different from the sizes of the collimators 260a, 260b, 260d, 260e, and 260f described above.

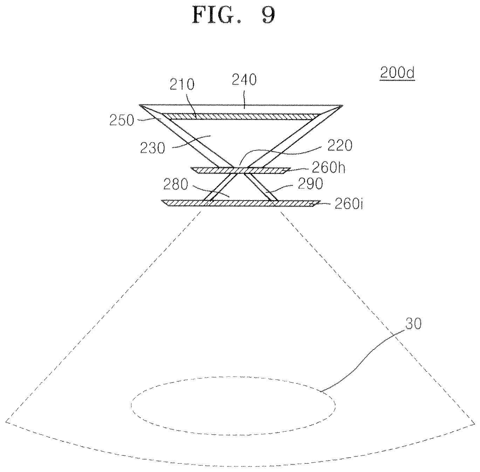

FIG. 9 is a view illustrating a radiation generator 200d according to another exemplary embodiment. Referring to FIG. 9, the radiation generator 200d includes a source 210 that includes a radioisotope and generates radiation, a first opening 220 through which a partial radiation irradiated in a specified direction passes, a converger 230 that converges the radiation irradiated from the source 210 into the first opening 220, and a diverger 290 that diverges the radiation that has passed through the first opening 220.

The radiation generator 200d further includes two collimators, namely, first collimator 260h and second collimator 260i. The first collimator 260h is disposed between the first opening 220 and the diverger 280, and the second collimator 260i is disposed between the diverger 280 and an object 30. Therefore, the first collimator 260h controls radiation passing through the first opening 220, and the second collimator 260i controls radiation passing through the diverger 280. For example, the first collimator 260h may control an irradiation or an interruption of radiation, and the second collimator 260i may control an amount of radiation or a radiation area. As described above, the collimator 260a or 260b described with reference to FIG. 2 or 4 may be applied as the first collimator 260h, and the collimator 260c, 260d, 260e, or 260f described with reference to FIGS. 4 through 7C may be applied as the second collimator 260i. However, the exemplary embodiment is not limited thereto, and thus one of the collimators 260a, 260b, 260c, 260d, 260e, and 260f may be applied as the first collimator 260h and the second collimator 260i.

Since the radiation generators 200a, 200b, 200c, and 200d described above have simple structures and perform simple operations, it is easy to design a radiation imaging apparatus including the radiation generator 200a, 200b, 200c, or 200d. In detail, a radiation generator that emits an electric field generates heat through electron collisions and thus additionally requires a cooling device. However, a radiation generator using a radioisotope does not need an additional cooling device. In particular, if the radiation generators 200a, 200b, 200c, and 200d are applied to an imaging apparatus, such as a CT apparatus, a rotation speed of a gantry is limited due to the installation of the cooling device in the related art. However, if a radioisotope according to the exemplary embodiment is used, a cooling device is not needed. Therefore, a rotation speed of a gantry does not need to be limited. Therefore, an object may be imaged within a short time. The radiation generators 200a, 200b, 200c, and 200d described above may be usefully applied to perform imaging on an object such as a heart of a patient

The radiation generators 200a, 200b, 200c, and 200d which use a radioisotope can generate radiation having different wavelength bands by changing the source 210.

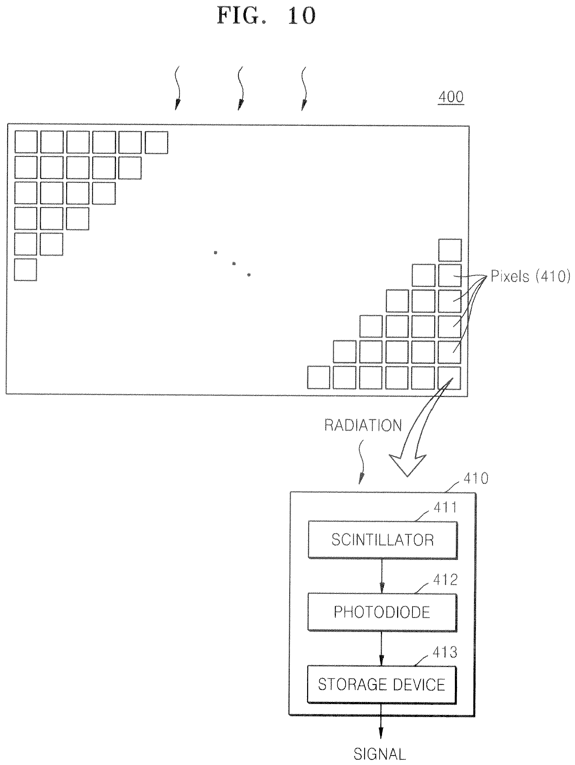

FIG. 10 is a schematic view illustrating the radiation detector 400 according to an illustrative example not being part of the invention. As shown in FIG. 10, the radiation detector 400 is synchronized with the radiation generator 200 in order to detect radiation and convert the detected radiation into an electrical signal.

The radiation detector 400 is formed of a plurality of pixels 410 that may respectively receive radiation. The pixels 410 may be formed in a two-dimensional (2D) array or in a one-dimensional array. Here, each of the pixels 410 may include light-receiving devices that receive radiation and convert the radiation into electrical signals. For example, the pixels 410 may include a scintillator 411, a photodiode (or a light diode) 412, and a storage device 413.

The scintillator 411 receives the radiation and outputs photons, in particular, visible photons, i.e., visible light, according to the received radiation. The photodiode 412 receives the photons output from the scintillator 411 and converts the photons into an electrical signal. The storage device 413 is electrically connected to the photodiode 412 and stores the electrical signal output from the photodiode 412. Here, the storage device 413 may be a storage capacitor or the like. Image processing is performed on the electrical signal stored in the storage device 413 of each of the pixels 410 in order to create a radiation image.

A radiation detector 400 may detect radiation by using a direct method of directly converting radiation into an electrical signal by using a photon counting detector.

Radiation irradiated from the radiation generator 200 penetrates the object 30, but can also be scattered by a medium such as air or the like. In particular, if scattered radiation is detected by the radiation detector 400, the scattered radiation becomes noise when generating an image.

Therefore, a radiation imaging apparatus according to an exemplary embodiment may include an anti-scatter grid that removes the scattered radiation.

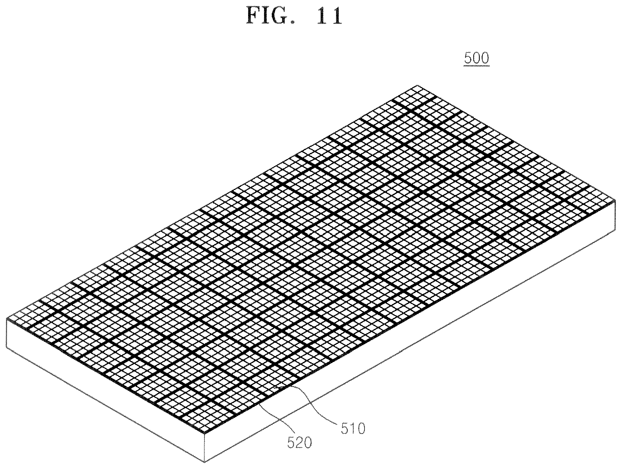

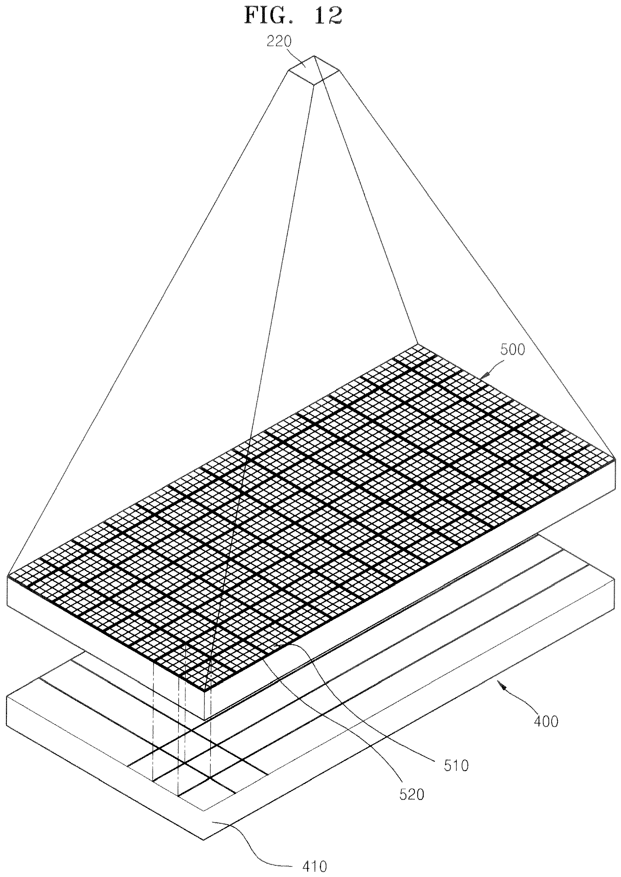

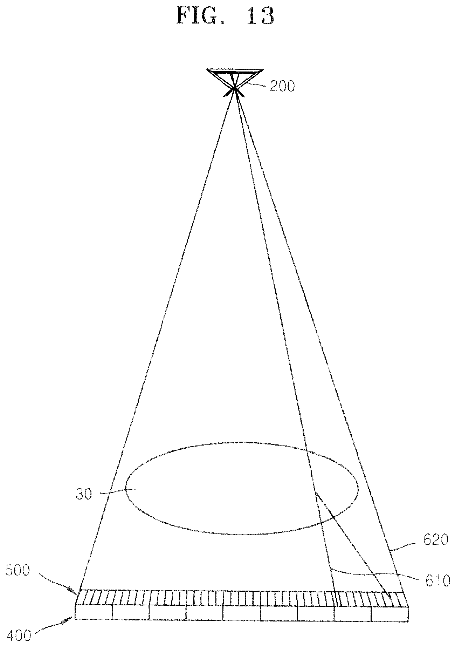

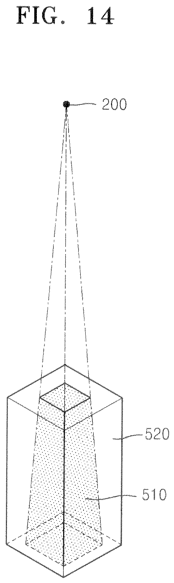

FIG. 11 is a view illustrating an anti-scatter grid 500 according to an illustrative example. FIG. 12 is a view illustrating a relationship between the anti-scatter grid 500 and a radiation detector 400. FIG. 13 is a schematic view illustrating a relationship between a radiation generator 200, the anti-scatter grid 500, and a radiation detector 400, according to an illustrative example. FIG. 14 is a view enlarging a radiation path and a barrier of FIG. 11. The radiation generator 200 of FIG. 13 may be the radiation generator 200 using a radioisotope or may be another type of radiation generator.

As shown in FIGS. 11 through 14, the anti-scatter grid 500 is disposed between an object 30 and the radiation detector 400. For example, the anti-scatter grid 500 may be disposed on a surface of the radiation detector 400 facing the object 30. Radiation is emitted from the radiation generator 200 toward the anti-scatter grid 500. Radiation irradiated from the radiation generator 200 is diverged from the radiation generator 200 toward the radiation detector 400. If radiation is irradiation from the radiation detector 400, the radiation is converged on the radiation generator 200. Therefore, if the anti-scatter grid 500 has a similar structure to a radiation pattern, the anti-scatter grid 500 may prevent radiation which is not-scattered from being removed.

In detail, the anti-scatter grid 500 includes a plurality of radiation paths 510 which pass radiation. The radiation which passes through the radiation paths 510 is incident onto the radiation detector 400. Barriers 520 divide the plurality of radiation paths 510 into two-dimensional (2D) type grid and prevent the emission of radiation between the radiation paths 510.

Cross-sections of the radiation paths 510 may be smaller than cross-sections of the pixels 410. Therefore, a plurality of radiation paths 510 may be two-dimensionally arrayed in an area corresponding to one of the pixels 410. Also, as shown in FIGS. 14, at least one of the radiation paths 510 may have a tapered shape. At least one of the radiation paths 510 may be converged on the radiation generator 200. For example, at least one of the radiation paths 510 may have a shape in which a cross-section widens from the radiation generator 200 toward the radiation detector 400. Radiation irradiated from the radiation generator 200 is gradually diverged from the radiation generator 200 toward the radiation detector 400. The radiation paths 510 may be empty spaces or may be filled with plastic or the like which the radiation can penetrate.

The barriers 520 may include a material that absorbs radiation. Therefore, radiation incident onto the barriers 520 is absorbed by the barriers 520. The barriers 520 may be formed of a material, such as silver (Ag), gold (Au), tungsten (W), molybdenum (Mo), or the like, having a high atomic number. Or, The barriers 520 may include a material that reflects radiation

The anti-scatter grid 500 may be manufactured by an inkjet method. For example, a first type of ink including a material absorbing radiation, such as a material having a high atomic number, is printed in an area corresponding to the barriers 520 on a substrate, and a second ink including a material transmitting radiation such as plastic is printed in the other area, i.e., in an area corresponding to the radiation paths 510. The above-described printing method is repeatedly performed to manufacture the anti-scatter grid 500 and then the substrate is removed from the anti-scatter grid 500. When the printing method is repeated, the radiation paths 510 are focused onto the radiation generator 200. The anti-scatter grid 500 is manufactured by using the printing method, and thus material costs and the amount of manufacturing time are reduced. Also, the anti-scatter grid 500 may be manufactured by 3D printing method.

The radiation paths 510 may be a space, but may be formed of a material that transmits radiation, such as plastic, to increase the strength of the anti-scatter grid 500. In other words, although the thickness of the barriers 520 are thin, a material filled in the radiation paths 510 keeps a shape of the anti-scatter grid 500. Therefore, a material having a weak mechanical strength, such as gold (Au), silver (Ag), copper (Cu), or the like, may be applied as a material of which the barriers 520 are formed.

Since the cross-sections of the radiation paths 510 are small, a plurality of radiation paths 510 may be two-dimensionally arrayed in an area corresponding to one of the pixels 410. The heights of the barriers 520 are proportional to the cross-sections of the radiation paths 510, and thus the cross-sections of the radiation paths 510 may be made small in order to make the height of the barriers 520 small. Therefore, since a height of the anti-scatter grid 500 is reduced, the anti-scatter grid 500 may be manufactured to improve a scattering removal effect and may have a small size. Also, since the plurality of radiation paths 510 are two-dimensionally arrayed in the area corresponding to one of the pixels 410, radiation which is scattered in various directions may be removed.

The anti-scatter grid 500 is disposed between the object 30 and the radiation detector 400 to allow radiation 610, which is not scattered, to be incident onto the radiation detector 400 and to allow radiation 620, which is scattered, to be removed by the anti-scatter grid 500.

The anti-scatter grid 500 is not restricted to removing radiation irradiated from the radiation generator 200 which uses a radioisotope. The anti-scatter grid 500 may be applied to remove radiation irradiated from a radiation generator or the like using an electric field.

As described above, according to the exemplary embodiment, a radiation generator having a simple structure is provided to easily design a radiation imaging apparatus.

Also, an anti-scatter grid according to the illustrative example removes radiation scattered in various directions. Further, the anti-scatter grid can be made small.

## Claims

1. A radiation generator comprising:
a radiation source (210) which comprises a radioisotope for generating radiation;
a first opening (220) configured to pass radiation among the generated radiation irradiated in a specified direction;
a converger (230) which has a cross-section that decreases toward the first opening and which is configured to converge the radiation irradiated from the radiation source into the first opening (220); and
a diverger (280) which has a cross-section that increases away from the first opening (220) and which diverges the radiation that has passed through the first opening (220);
**characterized in that**
the converger (230) comprises:
a first member (240) configured to block radiation which is irradiated in a direction opposite from an object (30); and
a second member (250) configured to surround the first member (240) and configured to guide radiation irradiated in a specified direction toward the object (30)
wherein the radiation source (210) is located inside a cavity of the converger (230) formed by the first member (240) and the second member (250).

2. The radiation generator of claim 1, wherein at least one of the first member (240) and the second member (250) comprises a material that absorbs the irradiated radiation.

3. The radiation generator of claim 1 or 2, further comprising:
a collimator (260a-i) configured to open the first opening (220) to control the radiation irradiated onto the object (30).

4. The radiation generator of claim 3, wherein the collimator (260b) is configured to horizontally move in a direction parallel with the first opening (220) in order to control an amount of radiation irradiated onto the object (30).

5. The radiation generator of claim 3, wherein the collimator (260c-f) is configured to rotate based on a central axis of the radiation generator in order to control an amount of radiation irradiated onto the object (30).

6. The radiation generator of claim 3, wherein the collimator (260b-f) comprises a second opening (270a-d) configured to pass radiation which has passed through the first opening (220).

7. The radiation generator of claim 3, wherein the collimator (260b, 260f) comprises a first area (261-263; 271-273) having a first radiation absorption amount with respect to the radiation and a second area (261-263; 271-273) having a second radiation absorption amount, different from the first radiation absorption amount, with respect to the radiation.

8. The radiation generator of any one of claims 1 to 7, wherein the radiation source (210) generates X-rays.

9. The radiation generator of any one of claims 1 to 8, wherein a divergence angle of the diverger (280) is smaller than a convergence angle of the converger (230).

## Patentansprüche

1. Strahlungsgenerator, mit:
einer Strahlungsquelle (210), die ein Radioisotop zur Erzeugung von Strahlung aufweist;
einer ersten Öffnung (220), die ausgebildet, Strahlung aus der erzeugten ausgestrahlten Strahlung in einer speziellen Richtung durchzulassen;
einer Bündelungseinheit (230), die einen Querschnitt hat, der sich zu der ersten Öffnung hin verkleinert, und die ausgebildet ist, die aus der Strahlungsquelle ausgestrahlte Strahlung in die erste Öffnung (220) zu bündeln; und
einer Zerstreuungseinheit (280), die einen Querschnitt hat, der sich von der ersten Öffnung (220) weggerichtet vergrößert, und die die Strahlung aufweitet, die die erste Öffnung (220) durchlaufen hat;
**dadurch gekennzeichnet, dass**
die Bündelungseinheit (230) aufweist:
ein erstes Element (240), das ausgebildet ist, Strahlung abzublocken, die in einer Richtung entgegengesetzt zu einem Objekt (30) ausgestrahlt wird; und
ein zweites Element (250), das ausgebildet ist, das erste Element (240) zu umgeben und Strahlung, die in einer speziellen Richtung ausgestrahlt wird, zu dem Objekt (30) zu führen,
wobei die Strahlungsquelle (210) in einer Aussparung der Bündelungseinheit (230) angeordnet ist, die durch das erste Element (240) und das zweite Element (250) gebildet ist.

2. Strahlungsgenerator nach Anspruch 1, wobei das erste Element (240) und/oder das zweite Element (250) ein Material aufweisen, das die ausgestrahlte Strahlung absorbiert.

3. Strahlungsgenerator nach Anspruch 1 oder 2, der ferner aufweist:
einen Kollimator (260a-i), der ausgebildet ist, die erste Öffnung (220) zur Steuerung der Strahlung, die auf das Objekt (30) gestrahlt wird, zu öffnen.

4. Strahlungsgenerator nach Anspruch 3, wobei der Kollimator (260b) ausgebildet ist, sich horizontal in einer Richtung parallel zu der ersten Öffnung (220) zu bewegen, so dass er eine Strahlungsmenge, die auf das Objekt (30) gestrahlt wird, steuert.

5. Strahlungsgenerator nach Anspruch 3, wobei der Kollimator (260c-f) ausgebildet ist, eine Drehung auf einer zentralen Achse des Strahlungsgenerators zur Steuerung einer Strahlungsmenge, die auf das Objekt (30) gestrahlt wird, auszuführen.

6. Strahlungsgenerator nach Anspruch 3, wobei der Kollimator (260b-f) eine zweite Öffnung (270a-d) aufweist, die ausgebildet ist, Strahlung, die durch die erste Öffnung (220) hindurchgetreten ist, durchzulassen.

7. Strahlungsgenerator nach Anspruch 3, wobei der Kollimator (260b, 260f) einen ersten Bereich (261-263; 271-273) mit einem ersten Strahlungsabsorptionsbetrag in Bezug auf die Strahlung und einen zweiten Bereich (261-263; 271-273) mit einem zweiten Strahlungsabsorptionsbetrag in Bezug auf die Strahlung, der sich von dem ersten Strahlungsabsorptionsbetrag unterscheidet, aufweist.

8. Strahlungsgenerator nach einem der Ansprüche 1 bis 7, wobei die Strahlungsquelle (210) Röntgenstrahlen erzeugt.

9. Strahlungsgenerator nach einem der Ansprüche 1 bis 8, wobei ein Divergenzwinkel der Zerstreuungseinheit (280) kleiner ist als ein Konvergenzwinkel der Bündelungseinheit (230).

## Revendications

1. Générateur de rayonnement comprenant :
une source de rayonnement (210) qui comprend un radioisotope pour une génération d'un rayonnement ;
une première ouverture (220) configurée pour laisser passer un rayonnement parmi le rayonnement généré irradié dans une direction spécifié ;
un convergeur (230) qui présente une section transversale qui diminue vers la première ouverture et qui est configuré pour faire converger le rayonnement irradié de la source de radiation vers la première ouverture (220) ; et
un divergeur (280) qui présente une section transversale qui augmente en s'éloignant de la première ouverture (220) et qui fait diverger le rayonnement qui a traversé la première ouverture (220) ;
**caractérisé en ce que**
le convergeur (230) comprend :
un premier élément (240) configuré pour bloquer un rayonnement qui est irradié dans une direction opposée depuis un objet (30) ; et
un deuxième élément (250) configuré pour entourer le premier élément (240) et configuré pour guider un rayonnement irradié dans une direction spécifiée vers l'objet (30)
dans lequel la source de rayonnement (210) est située à l'intérieur d'une cavité du convergeur (230) formée par le premier élément (240) et le deuxième élément (250).

2. Le générateur de rayonnement de la revendication 1, dans lequel au moins un du premier élément (240) et du deuxième élément (250) comprend un matériau qui absorbe le rayonnement irradié.

3. Le générateur de rayonnement de la revendication 1 ou 2, comprenant en outre :
un collimateur (260a-i) configuré pour ouvrir la première ouverture (220) pour contrôler le rayonnement irradié sur l'objet (30).

4. Le générateur de rayonnement de la revendication 3, dans lequel le collimateur (260b) est configuré pour se déplacer horizontalement dans une direction parallèle à la première ouverture (220) afin de contrôler une quantité de rayonnement irradié sur l'objet (30).

5. Le générateur de rayonnement de la revendication 3, dans lequel le collimateur (260c) est configuré pour tourner selon un axe central du générateur de rayonnement afin de contrôler une quantité de rayonnement irradié sur l'objet (30).

6. Le générateur de rayonnement de la revendication 3, dans lequel le collimateur (260b-f) comprend une deuxième ouverture (270a-d) configurée pour laisser passer un rayonnement qui a traversé la première ouverture (220).

7. Le générateur de rayonnement de la revendication 3, dans lequel le collimateur (260b, 260f) comprend une première zone (261-263 ; 271-273) présentant une première quantité d'absorption de rayonnement selon le rayonnement et une deuxième zone (261-263 ; 271-273) présentant une deuxième quantité d'absorption de rayonnement, différente de la première quantité d'absorption de rayonnement, selon le rayonnement.

8. Le générateur de rayonnement de l'une quelconque des revendications 1 à 7, dans lequel la source de rayonnement (210) génère des rayons X.

9. Le générateur de rayonnement de l'une quelconque des revendications 1 à 8, dans lequel un angle de divergence du divergeur (280) est inférieur à un angle de convergence du convergeur (230).
